(19) Europäisches Patentamt

European Patent Office

Office européen des brevets

(11) EP 0 856 206 B1

(12) **EUROPEAN PATENT SPECIFICATION**

(45) Date of publication and mention
of the grant of the patent:
**09.04.2003 Bulletin 2003/15**

(51) Int Cl.[7]: **G21F 9/00**, G21K 5/00

(21) Application number: **96936476.9**

(22) Date of filing: **16.10.1996**

(86) International application number:
**PCT/US96/16448**

(87) International publication number:
**WO 97/015104 (24.04.1997 Gazette 1997/18)**

(54) **METHOD OF ENHANCING RADIOACTIVITY DECAY**

VERFAHREN ZUR ERHÖHUNG DES RADIOAKTIVEN ZERFALLS

PROCEDE VISANT A AUGMENTER LA DECROISSANCE RADIOACTIVE

(84) Designated Contracting States:
**DE FR GB IT**

(30) Priority: **20.10.1995 US 5734 P**
**15.10.1996 US 729908**

(43) Date of publication of application:
**05.08.1998 Bulletin 1998/32**

(73) Proprietor: **Soloway, Sidney**
**Norwalk, CT 06851 (US)**

(72) Inventor: **Soloway, Sidney**
**Norwalk, CT 06851 (US)**

(74) Representative:
**Dempster, Benjamin John Naftel et al**
**Withers & Rogers,**
**Goldings House,**
**2 Hays Lane**
**London SE1 2HW (GB)**

(56) References cited:
**FR-A- 2 702 591          US-A- 4 815 447**
**US-A- 4 887 273          US-A- 5 003 544**
**US-A- 5 076 971          US-A- 5 274 689**

- **DATABASE WPI Section Ch, Week 8417 Derwent**
  **Publications Ltd., London, GB; Class K07, AN**
  **84-104925 XP002084690 -& JP 59 048698 A**
  **(DORYOKURO KAKUNENKYRO KAIHATSU), 19**
  **March 1984**
- **SOLOWAYA ET AL.: "High flux of appropriate**
  **photons for controlling radioactivity"**
  **TRANSACTIONS OF THE AMERICAN NUCLEAR**
  **SOCIETY, 1996, ANS,USA, vol. 75, page 20**
  **XP002084689**

## Description

The present invention relates to increasing rates of radioactive decay and particularly to a method of enhancing radioactive decay by bombarding a radioactive material with diverse energy photons.

Radioactive materials, while providing great benefits to society, often produce hazardous wastes and by-products which may remain dangerous for hundreds of thousands of years. Society has a need for improved techniques to dispose of the ever increasing amount of radioactive waste.

The length of time necessary for half of the atoms in a radioactive sample to decay is referred to as its "half-life." Although radioactive decay is a random process on the nuclear level, its macroscopic characteristics may be mathematically predicted and experimentally determined. For example, one common decay chain includes plutonium 239 ($_{92}Pu^{239}$) which decays to uranium 235 ($_{92}U^{235}$) with a half-life of 24,360 years. Eventually, $_{92}U^{235}$ decays to lead 207 ($_{82}Pb^{207}$), a stable particle. The process from $_{92}Pu^{239}$ of decay to lead 207 $_{82}Pb^{207}$ takes 12 steps. Another common decay chain includes decay from uranium 238 ($_{92}U^{238}$) to lead 206 ($_{82}Pb^{206}$) which takes 14 steps.

The half-life for the decay of a variety of radioactive materials is thousands of years. Such a long time period can cause long-lasting materials handling and storage problems with respect to these radioactive materials. Accordingly there is needed a method of accelerating the decay rate of radioactive materials beyond their normal rate of decay.

One such method has been disclosed in U.S. Patent No. 5,076,971 to Barker. Barker discloses accelerating radioactive decay by placing a radioactive material within the sphere or terminal of a Van de Graff generator and subjecting the material to a generator electric potential from 50 kilovolts (kV) to 500 kV, for at least a period of 30 minutes or more. Barker applies a negative electric potential to agitate the particles in the atomic system of a radioactive material. The large negative potential has the effect of lowering the energy barrier which retains the positively charged particles, such as alpha particles, within the nucleus. As a negative potential is placed upon the atomic nuclei, the rate of emission of alpha, beta, and gamma particles is increased. Barker suffers from being constrained to a location of a Van de Graff generator which must be specially constructed.

Accordingly, there is needed a method of accelerating the decay rate of radioactive materials beyond their normal rate of decay that is not subject to the limitations of the prior art.

In one aspect, the present invention relates to a method of increasing the rate of of radioactive decay of a radioactive isotope characterised by the steps of:

(a) providing a photon beam of x-ray or gamma-ray light having an effective energy and having an effective flux of photons sufficient to cause said increase in the rate of radioactive decay of the radioactive isotope when the photon beam is applied to said radioactive istotope;

(b) applying the photon beam to the radioactive isotope in order to contact the radioactive isotope with the photon beam, said photon beam having an energy level sufficient to enhance radioactivity decay of the radioactive isotope relative to the radioactive isotope's normal rate of decay, and said photon beam further having a flux level of the photon beam sufficient to accelerate a reduction in radioactivity of the radioactive isotope; and

(c) maintaining the photon beam being applied to the radioactive for an amount of time effective to cause said increase in the rate of radioactive decay of the radioactive isotope.

This typically will occur when the extent of emission of particles (e.g. alpha, beta and/or gamma particles) emitted from the radioactive sample is increased. The effective flux selected for the photon beam will be greater than the inherent flux of the radioactive isotope.

As used herein, x-ray is a generic term denoting electromagnetic photons within the x-ray spectrum which encompasses γ ray photons emitted from radioactive elements as well as photons emitted from diverse physics accelerators, such as the Synchrotron Light Source, along with photons emitted from more conventional x-ray equipment.

In yet another aspect, the present invention relates to a method of determining an effective photon energy and flux effective to increase radioactive decay of a radioactive isotope comprising the steps of:

(a) generating a beam of photons to provide a photon beam;

(b) placing a radioactive isotope in the photon beam;

(c) varying the energy and flux of the photon beam and selecting an effective flux that causes the radioactivity of the sample to decrease in an accelerated fashion, relative to the normal decrease in radioactivity of the sample over time. This typically will occur when the extent of emission of particles emitted from the radioactive sample is increased. The effective flux selected for the photon beam will be greater than the inherent flux of the radioactive isotope.

These and other aspects of the present invention will become readily apparent upon reading the following detailed description of the invention.

The accompanying drawings are included to provide a further understanding of the invention and are incorporated in and constitute a part of the specifications illustrating an embodiment of the invention and, together with the description, serve to explain the principles of the invention.

Figure 1 shows spectral density as a function of photon energy for beam X19B of the Brookhaven Synchrotron Light Source;
Figure 2 illustrates a configuration of a sample;
Figure 3 illustrates one embodiment according to the invention; and
Figure 4 illustrates another embodiment according to the invention;
Figure 5 illustrates still an example useful for understanding the invention;
Figure 6 illustrates placement of the samples according to Example 1; and
Figure 7 illustrates placement of the samples according to Example 2.

As used herein the term "stimulated emission" denotes methodology for increasing the rate of radioactive decay of a radioactive sample by bombarding the sample with photons to cause an increase in the emission of particles (e.g., alpha, beta and/or gamma particles from the radioactive sample, relative to the normal decay rate of the radioactive sample over time.

It is known that the half-lives of phosphorescent molecules or excited atoms are generally highly variable, but can be modified using the concept of stimulated emission. Stimulated emission as applied to atomic spectra results from exposing an excited atom to photons having an energy equal to the difference in energy between the atom's excited energy state and its ground energy state (or "ground state"). This energy difference is equal in energy to the energy of a photon which would be emitted by an excited atom during normal decay. Once stimulated by a photon of an appropriate energy level (which is equal to the difference between the exited and ground states), the energy of the atom will promptly drop to its ground state, and the atom will emit a photon of energy equal to the difference between the excited state and the ground state. Thus, for each stimulation of an atom by an externally-applied photon, two photons result, namely: the stimulating photon and the emitted photon. The above concept of stimulated emission as applied to atomic spectra provides the basis for the fundamental operation of lasers (light amplification by stimulated emission).

As applied to nuclear spectra, Mossbauer has described the absorption of resonant photons by stable (non-radioactive) nuclei. Mossbauer Spectroscopy and Its Applications, T.E. Cranshaw et al., 1985. The following equation describes the absorption of resonant photons in Mossbauer. spectroscopy:

$$R_{1\rightarrow2} = N_1 B_{1\rightarrow2} I(w) \tag{1}$$

where $R_{1\rightarrow2}$ represents a number of transitions from ground state 1 to excited state 2; $N_1$ represents a number of elements in ground state 1; $B_{1\rightarrow2}$ represents a "resonant capture cross section", which is larger than a non-resonant cross section, in state 1 in going to excited state 2 (for widely studied Mossbauer nuclei this is approximately $10^6$ barns); and I(w) represents a resonant flux of photons exciting ground state 1 (for Mossbauer nuclei this resonant bandwidth is approximately $10^{-2}$ to $10^{-6}$ electron-volts (eV)), The Feynman Lectures on Physics, Feynman et al., Addison-Wesley, 5th printing 1975. The "resonant capture cross-section" denotes the frequency range that causes the nucleus to be excited.

In accordance with the present invention, the present inventor has surprisingly discovered that the concepts embodied in stimulated emission as applied to atomic spectra apply to radioactive materials in nuclear spectra. Without wishing to be bound by any particular theory, the excited, metastable state 2 of atoms in a radioactive state have now been found to decay in a fashion analogous to an atom in an excited laser state when excited by a photon of an appropriate energy and causes it to drop to a lower energy state.

Prior to the present inventor's work, it was not considered that a radioactive nucleus would have an adequate resonant capture cross section. Accordingly, heretofore it was believed that a radioactive nuclei would not be amenable to "stimulated emission." Furthermore, it was believed that, even though only highly energetic photons of the order of 6 Mev to 20 MeV would be required, the capture cross section would still be too small to be effective to increase the decay rate.

A number of elements dropping from excited state 2 to ground state 1 is suitably described by the following equation in the atomic laser state:

$$R_{2\rightarrow1} = N_2 A_{2\rightarrow1} + N_2 B_{2\rightarrow1} I(w) \tag{2}$$

where $R_{2\rightarrow1}$ represents a number of transitions from excited, metastable state 2 to ground state 1; $N_2$ represents the number of elements in excited state 2; $A_{2\rightarrow1}$ represents a normal exponential decay factor in decaying from the metastable state 2 to the ground state 1; $B_{2\rightarrow1}$ represents a resonant capture cross section of state 2 when going to state 1; and I(w) represents a resonant flux of photons causing excited state 2 to transform to ground state 1. Feynman Lectures, referenced above.

The first term in Eq. (2) represents a normal dropping from excited state (2) to ground state 1. The second term of Eq. (2) represents the stimulated emission component beyond the normal rate. Also a photon could push a radioactive nucleus to a higher energy level so that its decay patterns would differ from that of the original nucleus.

Although, conventionally, radioactive elements have not been considered elements in a metastable state, the present inventor has applied the laser type analysis to radioactive decay within nuclear spectra.

In carrying out an experiment to confirm whether a laser type of analysis as conventionally applied to atomic spectra could be applied to radioactive materials, a special type of spectrophotometer would be particularly useful. Unfortunately, a specific spectrophotometer, having photons of a specific band width of, for example, $10^{-2}$ to $10^{-6}$ eV, and a high energy level of, for example, about 100 keV is not commercially available at the present time. Accordingly, the present inventor envisioned using a broad band width, high energy and flux photon beam to provide "stimulated emission" of radioactive target materials in order to accelerate the radioactive decay of the target using the analogy to a laser type analysis. Effective photons having an energy level of from about 10 eV to 10 MeV would be desirable however, to address all radioactive isotopes.

The present inventor located a suitable high energy light source having a broad band width, in the Brookhaven Synchrotron Light Source available at Brookhaven National Labs, Long Island, NY. This high energy light source has a photon energy range from 10 ev to 100 kev. The present inventor postulated that this Brookhaven Light Source would cause the normal rate of decay of $I^{125}$ to increase by a kind of "stimulated emission" as applied to nuclear spectra. The present inventor chose $I^{125}$ to test because of its ready availability and potential energy match to the source at Brookhaven. However, the general technique developed in accordance with the present invention is applicable to a wide variety of radioactive materials.

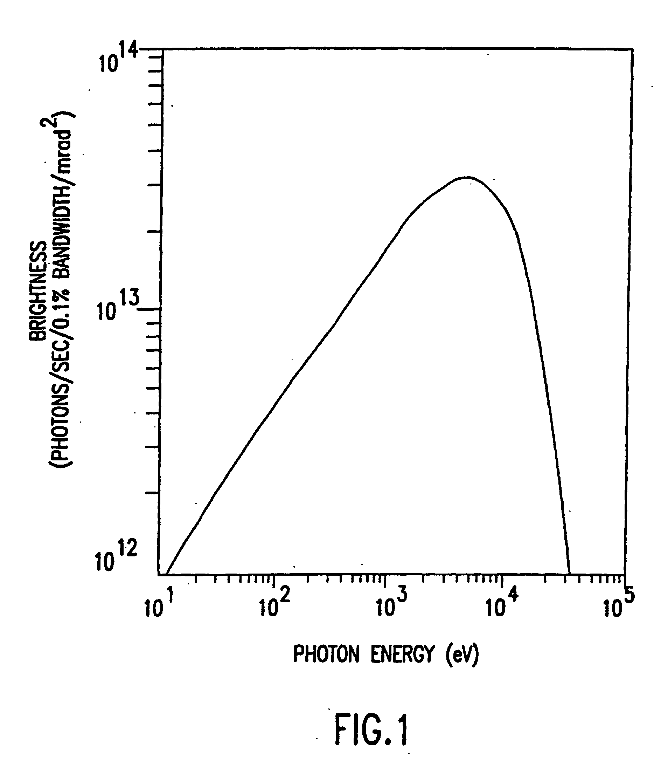

$I^{125}$'s radioactive half-life is 1430.4 hours, or approximately 2 months. $I^{125}$ emits a gamma ray photon of 35.5 keV while decaying. To evaluate "stimulated emission" as applied to nuclear spectra for this isotope, the present inventor used Brookhaven beam line X19B having an energy range of 10 eV to 50 keV as illustrated in curve X19B of Fig. 1 which shows spectral intensity as a function of photon energy. $I^{125}$ is a suitable target because its 35.5 keV gamma photon is within the energy range of beam X19B. At 35.5 keV, a central intensity of the beam is nearly $10^{12}$ photons/sec/0.1% band width/$mrad^2$ at 250 milliamps, which is adequately high to evaluate increasing radioactive decay. The present inventor expects fluxes greater than $10^8$ photon/sec/$cm^2$ to show appreciable radioactive decay increases.

Because resonant band widths are unavailable for radioactive nuclei, the present inventor approximated a nuclear resonant band width for $I^{125}$ at $10^{-3}$ to $10^{-6}$ eV, i.e., what the band width would be for a Mossbauer study as applied to stable nuclei.

Introducing a monochromator into the beam to select only gamma rays of 35.5 keV would require the sample to be 25 meters from the source, resulting in a very low photon flux. Without a monochromator a sample can be placed at a distance 3.5 meters from the light source, thereby providing an energy flux of about 2 x $10^8$ photons/sec/$10^{-5}$ eV beam width/$cm^2$ using a 250 milliampere beam. The absence of a monochromator prevents as clear a determination as might be desired of the photon energy that causes enhancement of the desired radioactivity reduction.

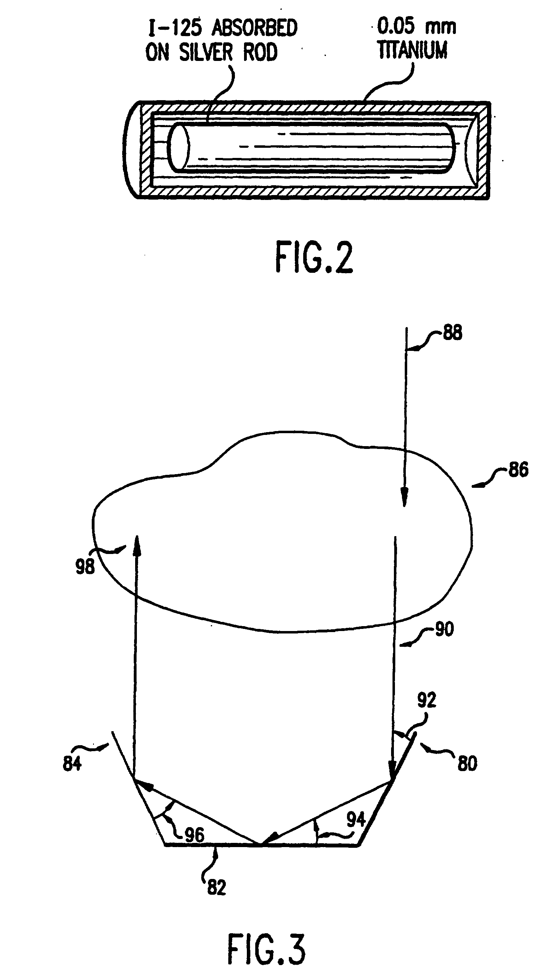

The present inventor selected samples made and distributed by Amersham Healthcare Med-Physics Inc. which are used to irradiate tumors. Its chemical structure is AgI, which is extremely stable chemically and physically, and is enclosed in a 0.05 mm titanium container as illustrated in Fig. 2. The radioactivity of the samples was about 0.03 mC before the experiment.

Based on extensive knowledge from atomic spectra, laser operation, and Mossbauer studies, an energy beam of x-rays initiates a kind of "stimulated emission," conventionally associated with atomic spectra, that causes a radioactive nucleus to drop to a lower energy state. The lower energy state may be a ground state whereby the nucleus would become non-radioactive or a different radioactive energy state requiring further "stimulated emission" to ultimately reach a ground non-radioactive state. Preferably, a white light x-ray source is used. More preferably, a band limited x-ray source is used having a bandwidth approximately equal to the radioactive resonant bandwidth as described below. Alternately, a resonant bandwidth could be approximated by using a Mossbauer non-radioactive resonant bandwidth.

For example, $I^{125}$ decays with a gamma ray of 35.5 keV. Mossbauer studies with non-radioactive, or stable nuclei estimate an energy bandwidth of $10^{-3}$ to $10^{-6}$ eV. Preferably, to reduce the radioactivity of an $I^{125}$ isotope, a suitable beam includes photons having an energy from about 10 eV to 100 keV, and a photon flux from about $10^9$ to $10^{13}$ photons/sec/$cm^2$ More preferably, a suitable beam includes photons having an energy from about 20 keV to 70 keV, and a photon flux from about $10^{10}$ to $10^{12}$ photons/sec/$cm^2$. Most preferably, a suitable beam includes photons having an energy from about 30 keV to 40 keV, and a photon flux from about $10^{10.5}$ to $10^{11.5}$ photons/sec/$cm^2$. The x-

rays could be generated by any source effective to produce x-rays having the desired characteristics identified above. For example, the x-ray source could be an x-ray beam, a Synchrotron Light Source, or any other appropriate radioactive source beam. Ideally, a narrow band spectrophotometer would be used to determine the energy and bandwidth that would be most effective in speeding up radioactive decay in advance of the irradiation.

Thus, the three components determining an effective x-ray beam for reducing the radioactivity of a radioactive isotope include x-ray energy, resonant bandwidth, and x-ray flux. Preferably, an effective x-ray beam includes x-rays with an energy about the energy decay value associated with the radioactive isotope, a resonant bandwidth from about $10^{-6}$ to $10^{-2}$ eV, and an x-ray flux greater than the natural flux of the radioactive isotope. More preferably, the beam includes photons with an energy that enhances radioactive decay, a resonant bandwidth determined for a radioactive isotope based on a Mossbauer analysis, and a flux greater than the natural flux of the radioactive isotope. Most preferably, an x-ray energy, resonant bandwidth, and flux as determined below.

Increasing the exposure time for a fixed flux, or increasing the flux for a fixed time will obviously lead to a greater radioactive reduction.

Although, this invention applies to all radioactive isotopes, some preferred isotopes include technetium-99, iodine-129, cesium-137, plus actinides such as plutonium, neptunium, and americium, cobalt-60, strontium-90, and combinations thereof.

The following method determines an effective x-ray energy, resonant bandwidth, and x-ray flux:

(a) assemble a high resolution Bragg scattering premonochromator spectrophotometer stage for x-rays to generate a very narrow bandwidth of x-rays from a Synchrotron Light Source or variable energy x-ray source, additional stages may be used to approach an x-ray energy bandwidth of $10^{-5}$ eV which would be preferred, although not critical, and if a narrow bandwidth is not available, then any bandwidth will suffice;

(b) place a radioactive sample whose radioactivity is to be reduced into an output beam of the assembly of step (a);

(c) vary the x-ray energy of the beam; and

(d) determine the x-ray energy where the reduction in radioactivity is greatest or where absorption generates a larger than normal peak in the forward direction and this will be the approximate effective x-ray energy.

In such a method, the x-ray flux for a $10^{-5}$ eV resonant bandwidth should be at least about $10^{9}$ photons/sec/$cm^{2}$ for a reasonable time for carrying out the sequence of individual measurements necessary for determining an effective x-ray energy for reducing radioactivity.

Considering a kind of "stimulated emission" as derived from laser studies to be a primary force in radioactivity reduction, the present inventor postulates that, for tritium, a 17.6 keV photon would be effective in accelerating its reduction in radioactivity, for strontium-90 a 610 keV photon, for tin (119 m) a 65 keV photon, the present inventor postulates that a 53 keV photon would enhance fission and reduce plutonium-239 half-life (several photon energies may be needed in the plutonium case to reduce it to a non-radioactive state), for polonium-210 a photon of 5.4 MeV, and for thorium-230 a photon of 4.767 MeV. These postulates should be backed up with a detailed technical analysis as described above to arrive at optimum photon energies.

Although Brookhaven was shown to be effective in enhancing radioactive decay, this synchrotron light source is extremely large and very expensive to build. Furthermore, there are very few such facilities worldwide. For most radioactive material, using Brookhaven is impractical.

Alternatively, a unique spectrophotometer could be used to obtain specific energy levels.

One could also use the Synchrotron Light Source in conjunction with a dispersion medium to obtain different energy levels spatially separated so that multiple materials could be bombarded simultaneously.

Other sources of photons exist, including x-ray tubes and γ-ray sources. Thus x-ray tubes of the right photon energy and photon flux could also be used. For example, in a hospital containing radioactive waste it would be convenient to haul the waste over to an appropriate x-ray unit, activate the unit, and quickly dispose of the radioactive hazard. Additionally, a portable x-ray source could also be developed for moving the enhancer to the source of the radioactivity if moving the radioactive material to the source is impractical or impossible. For example, accidentally exposed area or radioactive containment areas could have their decay enhanced without having to be taken apart or removed.

Another source of photons is a radioactive source. For example, $I^{125}$ is an x-ray source of 35.5 keV. Given an appropriate radioactive source that provides an appropriate flux of photons at the right energy for the material to be decayed also provides the desired energy for enhancing radioactive decay. Although, the radioactive material to be decayed itself is a source of photons of the appropriate energy, without the appropriate flux, enhancement effects are negligible.

As another alternative, a burst of x-rays may be employed, such as that provided by a pulsed x-ray source.

In one preferred embodiment, not all of the photons at the appropriate flux normally interact with the radioactive material. In another preferred embodiment, reflectors are employed to cause the photons to reflect back on the radioactive material. More specifically, X-rays do not reflect with conventional mirrors at 90 degrees because the x-rays penetrate the mirror. However, an x-ray striking a mirror at an angle less than 90 degrees, will reflect. For example, an apparatus for enhancing radioactive decay using x-rays includes a primary, a secondary, and a tertiary mirror. As herein embodied and illustrated in Fig. 3, an apparatus for enhancing radioactive decay using x-rays includes primary mirror 80, secondary mirror 82, and tertiary mirror 84. A radioactive material 86 is placed within x-ray beam 88. Some portion of beam 88 does not interact with radioactive material 86 and passes through radioactive material 86 to become beam 90. Beam 90 strikes primary mirror 80 at angle 92 which is less than 90 degrees and is reflected toward secondary mirror 82. Beam 90 strikes secondary mirror 82 at angle 94 also less than 90 degrees and is reflected toward tertiary mirror 84 striking at angle 96 which is less than 90 degrees. Tertiary mirror 84 reflects beam 90 back to radioactive material 86. Preferably, tertiary mirror 84 reflects beam 90 to a point 98 different from where beam 88 strikes radioactive material 86; however, tertiary mirror 84 could reflect beam 90 to any point on radioactive material 86, x-rays generated by the radioactive material would be reflected in the same way and cause x-ray amplification in the process.

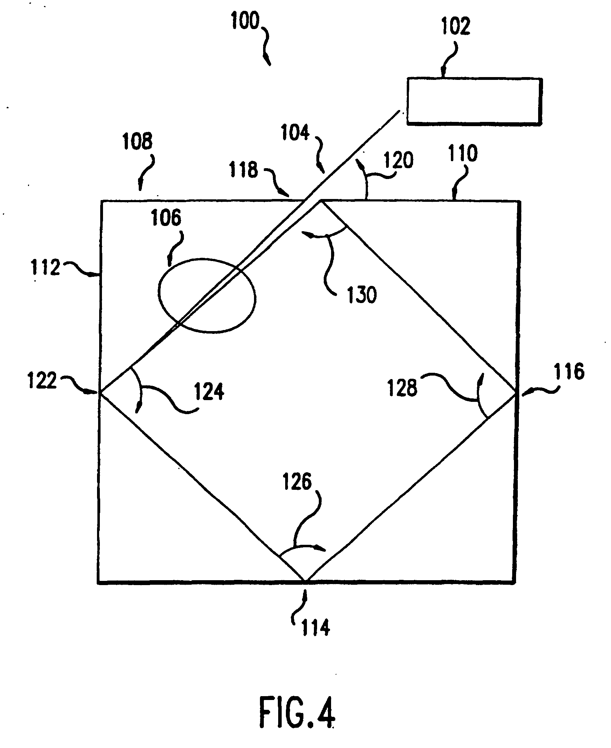

In another embodiment according to the present invention, an apparatus for enhancing the radioactivity decay of a radioactive sample includes an x-ray beam of appropriate energy and photon flux and a reflective chamber. As herein embodied and referring to Fig. 4, an apparatus for enhancing the radioactivity decay of a radioactive sample, denoted generally by the reference numeral 100, includes x-ray source 102, a beam 104, a radioactive material 106, and a reflective chamber denoted generally by reference numeral 108.

Reflective chamber 108 is effective to reflect x-ray photon generally in the same path. As herein embodied, reflective chamber 108 includes sides 110, 112, 114, and 116 forming a box. Each of sides 110, 112, 114, and 116 is effective in reflecting a substantial portion of photons. Side 110 includes aperture 118 effective to receive and pass through to the inside of reflective chamber 108 a beam of x-rays from x-ray source 102.

X-ray source 102 is positioned effective to supply a beam 104 of x-rays to aperture 118 at an angle 120 of 45 degrees. Beam 104 passes through aperture 118 and travels along a path effective to strike radioactive material 106. This portion of beam 104 which does not affect radioactive material 106 passes beyond radioactive material 106 to strike side 112 at midpoint 122 at an angle of 45 degrees. Beam 104 reflects from side 112 at 90 degrees from the pre-reflected path. The beam then travels and reflects from side 114 at midpoint 126 and then at side 116 at midpoint 128. Beam 104 then strikes side 110 at midpoint 130 and is reflected back to radioactive material 106. In this manner, beam 104 together with x-rays generated in source 106 are amplified in intensity. X-ray source 102 is controlled to prevent uncontrolled escalation of the intensity.

In the above described embodiment, the position of the radioactive material 106 is a design choice in that it may be placed anywhere within the reflective chamber 108 as long as it is within beam 104. Furthermore, the above configuration assumes an energy of x-rays most effectively reflected at 45 degrees from the reflective side. However, x-rays of differing energies are best reflected at differing angles. Accordingly, the number of reflective sides and angles of reflection would depend on the x-ray energy being reflected. For example, low energy x-rays, approximately 25 keV, best reflect when they strike a reflective surface at a large angle and accordingly would require few reflective surfaces to create a lasing effect. High energy x-rays, approximately 75 keV tend to reflect better at small angles and would thus require a larger number of reflective sides to create a lasing effect.

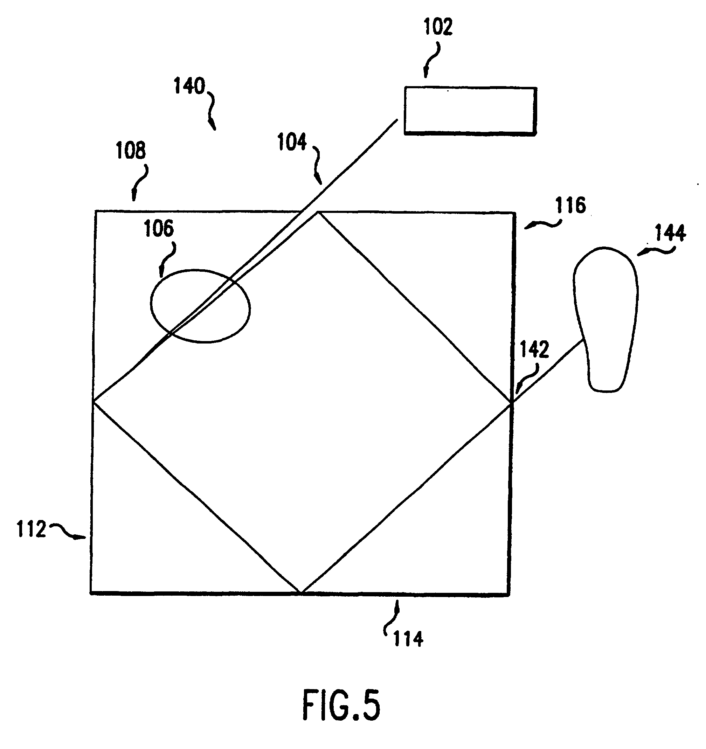

According to an example useful for understanding the invention, an x-ray laser is similar to apparatus described in Fig. 4, however, an aperture is provided to direct an amplified beam of x-rays to a particular source. According to the preferred embodiment an x-ray amplifier includes an x-ray source and a reflective chamber. As herein embodied and referring to Fig. 5, which is also an example useful for understanding the invention, an x-ray amplifier 140, includes x-ray source 102, and reflective chamber 108. Reference numerals corresponding to those of Fig. 5 have the same function and structure as defined with reference to Fig. 4. However, x-ray amplifier includes exit aperture 142 in side 116 allowing an amplified beam of x-rays to exit the reflective chamber 108 and strike position 144.

As with the embodiment described earlier, the number of particular configurations of reflective sides depends on the energy level of the x-rays to be reflected.

Such a system as described above, creates a lasing effect which results in a beam of x-rays within a narrower bandwidth, approximately $10^{-4}$ and lower, than can be conventionally obtained. Such an x-ray "laser" can lead to significant improvement in apparatus which use beams of x-rays. For example, x-ray beam lithography in semiconductor design fabrication is limited to certain minimum design constraints because of conventional x-ray beam bandwidths. An x-ray "laser" as made by the present invention, creates an x-ray beam with a much narrower bandwidth, which allows devices to be fabricated with much smaller device sizes.

The conventional x-ray tube is moderately broad in its band width. But, according to the present example, due to the reflective side arrangement and "stimulated emission", a much narrower energy bandwidth is possible than has been generated' heretofore.

In crystallography, this would provide a much narrower x-ray band width for determining the configuration of your crystal. In x-ray medicine you would also have the availability of doing a much more precise kind of analysis.

The method of enhancing radioactivity decay not in accordance with the invention can be applied to removing the radioactivity of radioactive materials given to patients by physicians in diagnosis and treatment of disease. By exposing a patient who has ingested, absorbed, or otherwise has internal radioactive material, to an appropriate photon energy and flux source, the radioactivity of the material may be eliminated or significantly reduced.

For example, as a prophetic example not in accordance with the invention, radioactive material is suitably administered to the patient, followed by the desired radiological analysis, and then the patient is exposed to an x-ray beam of an effective energy and flux to increase the radioactive decay of the radioactive material administered until the radioactivity in the patient is depleted.

Where a radioactive material has been concentrated in a tumor not in accordance with the invention, the radioactive decay can be enhanced.

By increasing the speed with which the radioactive material decays, the radioactive treatment is simultaneously sped up as well. An x-ray beam is applied to the radioactivity-containing tumor, having an effective energy and flux to enhance the radioactive decay for a predetermined time for an appropriate treatment, or to terminate the radioactivity of the radioactive material.

The following examples are intended to illustrate, but in no way limit the scope of, the present invention. All parts and percentages are by weight unless otherwise specified. As used herein, the term "eV" denotes election volts, "keV" denotes a thousand electron volts, the term "meV" denotes a million electron volts, and the term "mm" denotes millimeters.

EXAMPLE 1

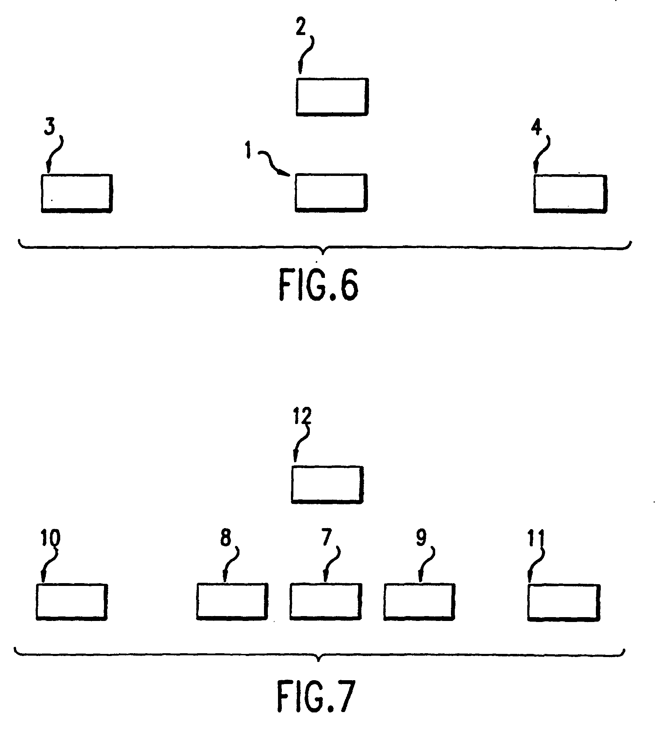

In a first example, four samples were exposed to Brookhaven beam X19B in a configuration illustrated in Fig. 6. Sample 1 is directly in the optimum photon flux. Sample 2 is slightly above the maximum flux. Samples 3 and 4 are located lateral to the flux maximum.

To account for effects due to temperature at the exposure point, the temperature at the location where sample 1 was to be placed was checked with the beam on and off. Temperature rise of less than 0.1°C was detected with beam on. The example was conducted at room temperature; however, temperature is not overly critical. Furthermore, the example was conducted at ambient air pressure. Pressure is also not overly critical to the success of the example.

The beam exposure over about 20 days for this experiment was recorded as a total of about 100 amp hours. Additionally, two samples (5,6) were used as controls, not exposed to beam X19B.

The counting apparatus for counting photons in the samples was an auto gamma 5000 Series Packard Gamma Counter with an aluminum liner so that the geometry of counting of all the samples could be compared reproducibly at the beginning and end of the experiment and with one another.

All the samples were measured for radioactivity at the beginning and end of the experiment. Their expected decay due to normal radioactive half-life decay was calculated and compared with the actual measured values of radioactivity after the experiment. The uncertainty in measurement is 0.5%.

The radioactivity of each of the two control samples was measured and found to be within 0.5% of one another after a time period equal to that during which the samples were exposed to beam X19B.

After having been exposed to beam X19B, sample 1 showed a decrease in radioactivity of 12.47% below an expected level after exposure.

Sample 2 showed a decrease in radioactivity of 2.17% below its calculated level. Samples 3 and 4 experienced scattered radiation from the main beam and were about 0.5% within their expected radioactivity level.

Additionally a "wipe test" was conducted immediately after the example. This wipe test was used to determine whether the decreased radioactivity was due to a loss of the material of the radioactive sample. Accordingly, a swab was used to wipe the area where the samples were located. The swab was then tested to determine whether it had any radioactivity. The swab after a sample wipe had a radioactivity of less than 10 picocuries; no detectable radioactivity release had occurred.

EXAMPLE 2

A second example was conducted one month later which exposed an additional 6 samples, samples 7 through 12. For the second trial the samples were placed as illustrated in Fig. 7. The results are summarized in Table 1. As can be seen from Table 1, sample 7, located directly in the beam, experienced an enhanced radioactivity decay of 13.8%.

Table 1

| Sample Number | % change from expected radioactivity level (error 0.5%) |
|---|---|
| 7 | -13.8 |
| 8 | -6.8 |
| 9 | -3.9 |
| 10 | -0.1 |
| 11 | 0.4 |
| 12 | -3.3 |
| Control 1 | 0.4 |
| Control 2 | -0.4 |

Additionally, the decay rate of the first 6 samples was checked to determine whether the first trial caused any long term effects to the samples while being stored between trials and is summarized in Table 2. As can be seen in Table 2, the normal radioactivity decay rate returned after the samples were no longer in the beam.

Table 2

| Sample | % change from expected radioactivity level (error 0.3%) |
|---|---|
| 1 | 0.1 |
| 2 | 0.0 |
| 3 | 0.2 |
| 4 | 0.1 |
| 5 (control) | 0.3 |
| 6 (control) | -0.3 |

The white light source used at Brookhaven is primarily limited to its energy range 10 eV to 50 keV. Other radioactive nuclei have different energy states and would require unique energies to reduce their radioactivity.

The white light from the Brookhaven Light Source Beam of 10 ev to 50 keV decreased the radioactivity of a sample of $I^{125}$ of radioactivity by 12% during a 100 ampere hour exposure. A repeat exposure on new samples caused a decrease of radioactivity for $I^{125}$ of 14%.

Although it is preferred that a bandwidth of $10^{-3}$ to $10^{-5}$ eV be used, a larger bandwidth would also be effective.

While the invention has been described above with reference to specific embodiments thereof, it is apparent that many changes, modifications and variations can be made without departing from the inventive concept disclosed herein. Accordingly, it is intended to embrace all such changes, modifications and variations that fall within the scope of the appended claims.

**Claims**

1.   A method of increasing the rate of of radioactive decay of a radioactive isotope (106) **characterised by** the steps of:

(a) providing a photon beam (104) of x-ray or gamma-ray light having an effective energy and having an effective flux of photons sufficient to cause said increase in the rate of radioactive decay of the radioactive isotope when the photon beam (104) is applied to said radioactive istotope (106);

(b) applying the photon beam to the radioactive isotope (106) in order to contact the radioactive isotope (106) with the photon beam (104), said photon beam (104) having an energy level sufficient to enhance radioactivity decay of the radioactive isotope (106) relative to the radioactive isotope's (106) normal rate of decay, and said photon beam (104) further having a flux level of the photon beam (104) sufficient to accelerate a reduction in radioactivity of the radioactive isotope (106); and

(c) maintaining the photon beam (104) being applied to the radioactive isotope (106) for an amount of time effective to cause said increase in the rate of radioactive decay of the radioactive isotope.

2. The method of claim 1 **characterised in that** the effective energy is greater than about 10 eV and the effective flux is greater than $10^7$ photon/sec/cm$^2$.

3. The method of claim 2 **characterised in that** the effective energy is an x-ray energy of from about 10 keV to about 10 MeV and the effective flux is greater than about $10^{10}$ photon/sec/cm$^2$.

4. The method according to claim 1 **characterized in that** the effective flux is greater than an inherent flux present in the radioactive isotope (106).

5. The method according to claim 1 **characterized in that** the step of providing the beam (104) comprises the steps of:

   (a) generating a beam (104) of photons to provide a photon beam;
   (b) placing a radioactive isotope sample (106) in the photon beam;
   (c) varying the energy and flux of the photon beam (104) and selecting an effective energy and flux that causes the radioactivity of the sample (106) to decrease in an accelerated fashion, relative to the normal decrease in radioactivity of the sample.

6. A method of determining an effective photon energy and flux effective to increase radioactive decay of a radioactive isotope **characterized by** the steps of:

   (a) generating a beam (104) of photons to provide a photon beam;
   (b) placing a radioactive isotope sample (106) in the photon beam;
   (c) varying the energy and flux of the photon beam and selecting an effective energy and flux that causes the radioactivity of the sample to decrease in an accelerated fashion, relative to the normal decrease in radioactivity of the sample.

7. The method of any preceding claim **characterised in that** said radioactive sample (106) comprises a composition selected from the group consisting of technetium, iodine, cesium-, plutonium, neptunium, americium, cobalt, and strontium isotopes and combinations thereof.

8. The method of any preceding claim **characterised in that** said photon energy level is greater than about 10 eV.

9. The method of any preceding claim **characterised in that** said energy level is between about 10 eV and about 10 MeV.

## Revendications

1. Un procédé pour augmenter la vitesse de désintégration radioactive d'un isotope radioactif (106), **caractérisé par** les étapes suivantes :

   (a) on fournit un faisceau de photons (104) de rayonnement consistant en rayons X ou en rayons gamma, ayant une énergie effective et ayant un flux de photons effectif suffisants pour produire cette augmentation de la vitesse de désintégration radioactive de l'isotope radioactif lorsque le faisceau de photons (104) est appliqué à l'isotope radioactif (106);
   (b) on applique le faisceau de photons à l'isotope radioactif (106) afin de mettre l'isotope radioactif (106) en contact avec le faisceau de photons (104), ce faisceau de photons (104) ayant un niveau d'énergie suffisant pour renforcer la désintégration radioactive de l'isotope radioactif (106), par rapport à la vitesse de désintégration normale de l'isotope radioactif (106), et ce faisceau de photons (104) ayant un niveau de flux du faisceau de photons (104) suffisant pour accélérer une réduction de la radioactivité de l'isotope radioactif (106); et
   (c) on maintient le faisceau de photons (104) appliqué à l'isotope radioactif (106) pendant une durée effective pour produire l'augmentation de la vitesse de désintégration radioactive de l'isotope radioactif.

2. Le procédé de la revendication 1, **caractérisé en ce que** l'énergie effective est supérieure à environ 10 eV et le flux effectif est supérieur à $10^7$ photons/s/cm$^2$.

3. Le procédé de la revendication 2, **caractérisé en ce que** l'énergie effective est une énergie de rayons X allant d'environ 10 keV à environ 10 MeV et le flux effectif est supérieur à environ $10^{10}$ photons/s/cm$^2$.

**4.** Le procédé selon la revendication 1, **caractérisé en ce que** le flux effectif est supérieur à un flux inhérent présent dans l'isotope radioactif (106).

**5.** Le procédé selon la revendication 1
**caractérisé en ce que** l'étape de fourniture du faisceau (104) comprend les étapes suivantes :

(a) on génère un faisceau (104) de photons pour fournir un faisceau de photons;
(b) on place un échantillon d'isotope radioactif (106) dans le faisceau de photons;
(c) on fait varier l'énergie et le flux du faisceau de photons (104) et on sélectionne une énergie et un flux effectifs qui provoquent une diminution accélérée de la radioactivité de l'échantillon (106), par rapport à la diminution de radioactivité normale de l'échantillon.

**6.** Un procédé de détermination d'une énergie et un flux de photons effectifs pour augmenter la désintégration radioactive d'un isotope radioactif, **caractérisé par** les étapes suivantes :

(a) on génère un faisceau (104) de photons pour produire un faisceau de photons;
(b) on place un échantillon d'isotope radioactif (106) dans le faisceau de photons;
(c) on fait varier l'énergie et le flux du faisceau de photons et on sélectionne une énergie et un flux effectifs qui provoquent une diminution accélérée de la radioactivité de l'échantillon, par rapport à la diminution de radioactivité normale de l'échantillon.

**7.** Le procédé de l'une quelconque des revendications précédentes, **caractérisé en ce que** l'échantillon radioactif (106) a une composition sélectionnée dans le groupe consistant en isotopes de technétium, iode, césium, plutonium, neptunium, américium, cobalt et strontium, et des combinaisons de ceux-ci.

**8.** Le procédé de l'une quelconque des revendications précédentes, **caractérisé en ce que** le niveau d'énergie des photons est supérieur à environ 10 eV.

**9.** Le procédé de l'une quelconque des revendications précédentes, **caractérisé en ce que** le niveau d'énergie est compris entre environ 10 eV et environ 10 MeV.

**Patentansprüche**

**1.** Ein Verfahren zum Erhöhen der radioaktiven Zerfallsrate eines radioaktiven Isotops (106), **gekennzeichnet durch** die Schritte:

(a) Bereitstellen eines Photonenstrahls (104) aus Röntgen- oder Gammalicht, der eine effektive Energie und einen effektiven Photonenfluss aufweist, die ausreichen, um das Erhöhen der radioaktiven Zerfallsrate des radioaktiven Isotops zu bewirken, wenn der Photonenstrahl (104) auf das radioaktive Isotop (106) angewendet wird;
(b) Anwenden des Photonenstrahls auf das radioaktive Isotop (106), um das radioaktive Isotop (106) mit dem Photonenstrahl (104) in Kontakt zu bringen, wobei der Photonenstrahl (104) ein Energieniveau aufweist, das ausreicht, um das Abklingen des radioaktiven Isotops (106) relativ zu der normalen Zerfallsrate des radioaktiven Isotops (106) zu erhöhen, und wobei der Photonenstrahl (104) weiterhin ein Flussniveau des Photonenstrahls (104) aufweist, das ausreicht, um eine Reduzierung der Radioaktivität des radioaktiven Isotops (106) zu beschleunigen; und
(c) Aufrechterhalten des auf das radioaktive Isotop (106) angewendeten Photonenstrahls (104) für eine Zeitdauer, die wirksam ist, um den Anstieg in der radioaktiven Zerfallsrate des radioaktiven Isotops zu verursachen.

**2.** Das Verfahren nach Anspruch 1, **dadurch gekennzeichnet, dass** die effektive Energie größer als ungefähr 10 eV und der effektive Fluss größer als $10^7$ Photonen/sec/cm$^2$ ist.

**3.** Das Verfahren nach Anspruch 2, **dadurch gekennzeichnet, dass** die effektive Energie eine Röntgenenergie von ungefähr 10 keV bis ungefähr 10 MeV und der effektive Fluss größer als ungefähr $10^{10}$ Photonen/sec/cm$^2$ ist.

**4.** Das Verfahren nach Anspruch 1, **dadurch gekennzeichnet, dass** der effektive Fluss größer als ein in dem radioaktiven Isotop (106) vorliegender inhärenter Fluss ist.

5. Das Verfahren nach Anspruch 1, **dadurch gekennzeichnet, dass** der Schritt des Bereitstellens des Strahls (104) die Schritte umfasst:

(a) Erzeugen eines Strahls (104) aus Photonen, um einen Photonenstrahl bereitzustellen;
(b) Platzieren einer radioaktiven Isotopenprobe (106) in den Photonenstrahl;
(c) Variieren der Energie und des Flusses des Photonenstrahls (104) und Auswählen einer effektiven Energie und eines effektiven Flusses, der bewirkt, dass die Radioaktivität der Probe (106) in einer relativ zu der normalen Abnahme der Radioaktivität der Probe beschleunigten Weise abnimmt.

6. Ein Verfahren zum Bestimmen einer effektiven Photonenenergie und eines effektiven Photonenflusses, die wirksam sind, den radioaktiven Zerfall eines radioaktiven Isotops zu erhöhen, **gekennzeichnet durch** die Schritte:

(a) Erzeugen eines Strahls (104) aus Photonen, um einen Photonenstrahl bereitzustellen;
(b) Platzieren einer radioaktiven Isotopenprobe (106) in den Photonenstrahl;
(c) Variieren der Energie und des Flusses des Photonenstrahls und Auswählen einer effektiven Energie und eines effektiven Flusses, der bewirkt, dass die Radioaktivität der Probe in einer relativ zu der normalen Abnahme der Radioaktivität der Probe beschleunigten Weise abnimmt.

7. Das Verfahren nach irgendeinem vorhergehenden Anspruch, **dadurch gekennzeichnet, dass** die radioaktive Probe (106) eine Zusammensetzung aufweist, die aus der Gruppe ausgewählt wurde, welche aus Technetium-, Iod-, Cäsium-, Plutonium-, Neptunium-, Americium-, Kobalt- und Strontium-Isotopen und Kombinationen hiervon besteht.

8. Das Verfahren nach irgendeinem vorhergehenden Anspruch, **dadurch gekennzeichnet, dass** das Photonenenergieniveau größer als ungefähr 10 eV ist.

9. Das Verfahren nach irgendeinem vorhergehenden Anspruch, **dadurch gekennzeichnet, dass** das Energieniveau zwischen ungefähr 10 eV und ungefähr 10 MeV liegt.

FIG.1

I-125 ABSORBED ON SILVER ROD    0.05 mm TITANIUM

FIG.2

FIG.3

FIG.4

FIG.5

2

3    1    4

FIG.6

12

10    8    7    9    11

FIG.7